# EUROPEAN PATENT APPLICATION

(11) **EP 4 768 602 A1**
(43) Date of publication of application: **01.07.2026**
(21) Application number: 23950921.9
(22) Date of filing: 03.11.2023
(51) Int. Cl.: C12Q 1/6886, G01N 33/574

(54) **COMPOSITION FOR DIAGNOSIS OF RADIORESISTANCE OF LUNG CANCER OR FOR PROGNOSIS OF LUNG CANCER**

(30) Priority: 25.08.2023 KR 20230112011
(71) Applicant: Korea Institute of Radiological & Medical Sciences, Seoul 01812 (KR)
(72) Inventor: SONG, Jie Young, Seoul 07324 (KR); PARK, Jeong In, Seoul 02562 (KR); JUNG, Seung Youn, Seoul 01817 (KR); SONG, Kyung Hee, Namyangju-si, Gyeonggi-do 12141 (KR); LEE, Dong Hyeon, Gunpo-si, Gyeonggi-do 15810 (KR); LEE, Jee Yong, Seoul 06194 (KR); AHN, Jiyeon, Seoul 01721 (KR); PARK, Jong Kuk, Seoul 05262 (KR); HWANG, Sang Gu, Seoul 01727 (KR)
(74) Representative: Cabinet Beau de Loménie
(86) International application number: PCT/KR2023/017587
(87) International publication number: WO 2025/048046

(57) **Abstract**

In the present invention, the mRNA of the genes VTN, CXCL5, NEDD4, LCN2, SYTL4, TNFR2, and SERPING1, or the proteins encoded by these genes, exhibit differential expression levels in radioresistant lung cancer cells compared to control lung cancer cells and have a close correlation with a decrease in the survival rate of lung cancer patients. Accordingly, this preparation for measuring the expression levels of the mRNA of the genes selected from the group consisting of VTN, CXCL5, NEDD4, LCN2, SYTL4, TNFR2, and SERPING1 of the present invention or the expression levels of the proteins encoded by the genes can effectively diagnose radiation resistance in lung cancer or prognose radiation therapy for lung cancer, whereby it is possible to determine whether to perform radiation therapy and the optimal dose of radiation therapy, thus improving the efficiency and therapeutic outcomes of radiation therapy for lung cancer.

## Description

### [Technical Field]

### CROSS-REFERENCE TO RELATED APPLICATION

This application claims the benefit of priority to Korean Patent Application No. 10-2023-0112011, filed in the Korean Intellectual Property Office on August 25, 2023, the entire contents of which are incorporated herein by reference.

### [Technical Field]

The present disclosure relates to a composition for diagnosing radioresistance of lung cancer, and an application thereof.

### [Background Art]

Cancer treatment methods include surgical operation, chemical drug therapy, and radiotherapy, and recently, the importance of radiotherapy has been further increasing. Cases, in which tumors have been efficiently cured only by radiotherapy when surgical operation is difficult, have been reported, and tumor treatment methods using radiation have also been developing year by year, and accordingly, radiotherapy has been established as a method capable of efficiently treating tumors in the body without causing special pain or resistance to patients. Statistically, about 30-50% of cancer patients receive radiotherapy at some point during their treatment period. Because the number of cancer patients who receive radiotherapy has been increasing every year, the importance of radiotherapy in cancer treatment is also increasing.

Radiotherapy is commonly used either alone or in combination with chemotherapeutic agents to treat various types of cancer. However, the effectiveness of radiotherapy varies depending on the characteristics of the cancer, the patient, and whether the radiation is combined with other treatments. In general, cancers, on which radiotherapy is widely performed, include cervical cancer, pharyngeal cancer, lung cancer, brain cancer, and breast cancer. Although these types of cancer are major targets of radiotherapy, poor responses to radiotherapy frequently occur, and thus, strategies to improve treatment efficiency are required. In addition, although radiotherapy may be well performed and show a good initial response, recurrence frequently occurs, and thus, establishing countermeasures against recurrent cancer is also important for improving the efficiency of radiotherapy.

Furthermore, acquisition of radioresistance by cancer cells and damage to normal tissues during high-dose radiotherapy have been pointed out as problems that reduce the efficiency of radiotherapy, and thus, research to enhance the efficiency of radiotherapy is required. Currently, radiotherapy, together with surgical operation or chemical drug therapy, is an effective cancer treatment method and is used for the purpose of killing cancer cells by applying radiation to the cancer tissue of a patient.

Although there are differences in the sensitivity of cancer cells to radiotherapy, by developing a technique capable of predicting individual sensitivity to radiation before radiotherapy, it would be possible to treat cancer by adjusting the radiation dose for each individual, thereby providing the most suitable radiotherapy for each patient.

Meanwhile, in recent years, studies on companion diagnostics, which refer to approved diagnostics that may select appropriate targeted anticancer drugs and treatment methods based on systematic analysis results of individual patient factors, have been actively conducted. Because companion diagnostics may provide clear clinical evidence for prescriptions based on a physician's diagnosis and suggest appropriate treatment methods for patients, they can not only improve the efficiency of cancer treatment but also reduce the misuse or overuse of targeted anticancer drugs, thereby contributing to the financial soundness of the national health insurance system. Currently, the companion diagnostics market is growing in therapeutic fields such as breast cancer, lung cancer, colorectal cancer, gastric cancer, and melanoma, and in particular, the fields of breast cancer and lung cancer are expected to drive market growth.

Korean Patent Application No. 10-2021-0138525 discloses a technology of measuring an mRNA expression level of exosomal miR-151a-3p to be used in diagnosing radiotherapy sensitivity of cancer patients or predicting a radiotherapy prognosis, but does not suggest data regarding lung-cancer-cell specificity.

Furthermore, Korean Patent Application No. 10-2018-0143493 discloses a technology of diagnosing radioresistant lung cancer or pancreatic cancer by measuring an expression level of PMVK protein or mRNA, but does not suggest a technology capable of more accurately diagnosing radioresistant lung cancer by using multiple markers.

Under this background, the inventors of the present disclosure conducted research on radioresistance biomarkers and discovered novel biomarkers for diagnosing radioresistant lung cancer. The biomarkers are expected to be usefully applicable to lung-cancer treatment, because they may predict radioresistance or the prognosis of radiotherapy.

### [Prior art documents]

### [Patent documents]

Korean Patent Application No. 10-2021-0138525
Korean Patent Application No. 10-2018-0143493

### [Disclosure]

### [Technical Problem]

An aspect of the present disclosure provides a composition for diagnosing radioresistance of lung cancer.

An aspect of the present disclosure also provides a kit for diagnosing radioresistance of lung cancer.

Another aspect of the present disclosure also provides a method for providing information for diagnosing radioresistance of lung cancer.

An aspect of the present disclosure also provides a composition for predicting a prognosis of radiotherapy for lung cancer.

An aspect of the present disclosure also provides a kit for predicting a prognosis of radiotherapy for lung cancer.

An aspect of the present disclosure also provides a method for providing information for predicting a prognosis of radiotherapy for lung cancer.

### [Technical Solution]

An aspect of the present disclosure provides a composition for diagnosing radioresistance of lung cancer, comprising an agent for measuring expression levels of mRNA of two or more genes selected from the group consisting of VTN, CXCL5, NEDD4, LCN2, SYTL4, TNFR2, and SERPING1, or proteins encoded by two or more of the genes.

Furthermore, another aspect of the present disclosure provides a kit for diagnosing radioresistance of lung cancer, which comprises the composition for diagnosing radioresistance of lung cancer.

Furthermore, another aspect of the present disclosure provides a method for providing information for diagnosing radioresistance of lung cancer, comprising (a) measuring, in a sample isolated from a subject, expression levels of mRNA of two or more genes selected from the group consisting of VTN, CXCL5, NEDD4, LCN2, SYTL4, TNFR2, and SERPING1, or proteins encoded by two or more of the genes, and (b) comparing the expression levels of the mRNA or proteins with expression levels of mRNA or proteins of a normal control group.

Furthermore, another aspect of the present disclosure provides a composition for predicting a prognosis of radiation therapy of lung cancer, comprising an agent for measuring expression levels of mRNA of two or more genes selected from the group consisting of VTN, CXCL5, NEDD4, LCN2, SYTL4, TNFR2, and SERPING1 genes, or expression levels of proteins encoded by two or more of the genes.

Furthermore, another aspect of the present disclosure provides a kit for predicting a prognosis of radiotherapy for lung cancer, which comprises the composition for predicting a prognosis of radiotherapy for lung cancer.

Furthermore, another aspect of the present disclosure provides a method for providing information for predicting a prognosis of radiotherapy for lung cancer, comprising (a) measuring, in a sample isolated from a subject, expression levels of mRNA of two or more genes selected from the group consisting of VTN, CXCL5, NEDD4, LCN2, SYTL4, TNFR2, and SERPING1, or proteins encoded by two or more of the genes, and (b) comparing the expression levels of the mRNA or proteins with expression levels of mRNA or proteins of a normal control group.

Hereinafter, the present disclosure will be described in detail.

### 1. Composition for Diagnosing Radioresistance of Lung Cancer or Predicting a Prognosis of Radiotherapy for Lung Cancer

An aspect of the present disclosure provides a composition for diagnosing radioresistance of lung cancer.

Furthermore, another aspect of the present disclosure provides a composition for predicting a prognosis of radiotherapy for lung cancer.

The composition for diagnosing radioresistance of lung cancer according to the present disclosure includes, as an active ingredient, an agent for measuring expression levels of mRNA of two or more genes selected from the group consisting of VTN, CXCL5, NEDD4, LCN2, SYTL4, TNFR2, and SERPING1, or proteins encoded by two or more of the genes.

Furthermore, the composition for predicting a prognosis of radiotherapy for lung cancer according to the present disclosure includes, as an active ingredient, an agent for measuring expression levels of mRNA of two or more genes selected from the group consisting of VTN, CXCL5, NEDD4, LCN2, SYTL4, TNFR2, and SERPING1, or proteins encoded by two or more of the genes.

It is known that VTN (vitronectin, NCBI Gene ID: 7448) protein is a hemopexin-family glycoprotein synthesized in the liver, promotes cell adhesion and migration by binding to integrin αVβ3 to connect cells to an extracellular matrix, and is involved in hemostasis as a component of platelets.

In the present disclosure, the VTN protein refers to not only the wild-type VTN protein consisting of the amino acid sequence of SEQ ID No: 1, but also equivalents having the same or similar function or activity as the wild-type VTN protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 1.

The CXCL5 (C-X-C motif chemokine ligand 5, NCBI gene ID: 6374) is a small cytokine belonging to the CXC chemokine family, and is known to be produced by stimulation with the inflammatory cytokines interleukin-1 and tumor necrosis factor-alpha, and to regulate neutrophil homeostasis by suppressing type II interferon-gamma.

In the present disclosure, the CXCL5 protein refers to not only the wild-type CXCL5 protein consisting of the amino acid sequence of SEQ ID No: 2, but also equivalents having the same or similar function or activity as the wild-type CXCL5 protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 2.

Neural precursor cell expressed developmentally down-regulated protein 4 (NEDD4) (NCBI Gene ID: 4734) protein is an E3 ubiquitin ligase, and it is known that the protein regulates activities of membrane proteins, such as ion channels and membrane receptors, through ubiquitination and endocytosis.

In the present disclosure, the NEDD4 protein refers to not only the wild-type NEDD4 protein consisting of the amino acid sequence of SEQ ID No: 3, but also equivalents having the same or similar function or activity as the wild-type NEDD4 protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 3.

The lipocalin 2 (LCN2) (NCBI Gene ID: 3934) protein, also referred to as neutrophil gelatinase-associated lipocalin (NGAL), is known to be involved in innate immunity by sequestering iron and preventing its utilization by bacteria, thereby restricting their growth.

In the present disclosure, the LCN2 protein refers to not only the wild-type LCN2 protein consisting of the amino acid sequence of SEQ ID No: 4, but also equivalents having the same or similar function or activity as the wild-type LCN2 protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 4.

The synaptotagmin like 4 (SYTL4) (NCBI Gene ID: 94121) protein has an N-terminal Rab27-binding domain and a C-terminal tandem C2 domain, and is known to bind to Rab GTPase to participate in intracellular membrane trafficking, as well as to mediate the release of lytic granules into the cell and regulate the secretion of pancreatic and pituitary hormones.

In the present disclosure, the SYTL4 protein refers to not only the wild-type SYTL4 protein consisting of the amino acid sequence of SEQ ID No: 5, but also equivalents having the same or similar function or activity as the wild-type SYTL4 protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 5.

The tumor necrosis factor receptor 2 (TNFR2) (NCBI Gene ID: 7133) protein, also referred to as tumor necrosis factor receptor superfamily member 1B (TNFRSF1B), is one of the membrane receptors that bind to TNFα, and is known to induce transcription of genes related to cell survival, growth, and differentiation.

In the present disclosure, the TNFR2 protein refers to not only the wild-type TNFR2 protein consisting of the amino acid sequence of SEQ ID No: 6, but also equivalents having the same or similar function or activity as the wild-type TNFR2 protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 6.

The serpin family G member 1 (SERPING1) (NCBI Gene ID: 710) protein is a protease inhibitor belonging to the serpin superfamily. It is also referred to as the C1 esterase inhibitor (C1-INH) and is known to regulate physiological pathways, including complement activation, blood coagulation, fibrinolysis, and kinin generation, by inhibiting the activation of the C1 complex.

In the present disclosure, the SERPING1 protein refers to not only the wild-type SERPING1 protein consisting of the amino acid sequence of SEQ ID No: 7, but also equivalents having the same or similar function or activity as the wild-type SERPING1 protein, although their sequences are partially different, and the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 7.

The composition for diagnosing radioresistance of lung cancer according to the present disclosure may include, as an active ingredient, an agent for measuring expression levels of mRNA of two genes, three genes, four genes, five genes, six genes, or seven genes selected from the group consisting of VTN, CXCL5, NEDD4, LCN2, SYTL4, TNFR2, and SERPING1, or proteins encoded by the genes.

Furthermore, the composition for predicting a prognosis of radiotherapy for lung cancer according to the present disclosure may include, as an active ingredient, an agent for measuring expression levels of mRNA of two genes, three genes, four genes, five genes, six genes, or seven genes selected from the group consisting of VTN, CXCL5, NEDD4, LCN2, SYTL4, TNFR2, and SERPING1, or proteins encoded by the genes.

In a specific embodiment of the present disclosure, it was identified that the mRNA of a gene selected from the group consisting of VTN, CXCL5, NEDD4, LCN2, SYTL4, TNFR2, and SERPING1, or a protein encoded by the gene, has a differential expression level in radioresistant lung-cancer cells compared with control lung-cancer cells, and has a close correlation with decreased survival rates of lung-cancer patients.

Accordingly, an agent for measuring expression levels of mRNA of one or two or more genes selected from the group consisting of VTN, CXCL5, NEDD4, LCN2, SYTL4, TNFR2, and SERPING1, or proteins encoded by one or two or more of the genes, may diagnose the radioresistance of lung cancer and predict the prognosis of lung-cancer radiotherapy more effectively than mRNA and/or proteins of other genes and their combinations, and through this, the efficiency and therapeutic outcome of radiotherapy for lung cancer may be improved by determining whether to perform radiotherapy and by setting an optimal radiation dose for radiotherapy.

Specifically, in the present disclosure, the VTN, NEDD4, CXCL5, LCN2, SYTL4, TNFR2, and SERPING1 genes may be genes whose expression is upregulated when lung cancer has radioresistance, whose expression is upregulated by radiation exposure, and whose upregulated expression is associated with decreased survival rates of lung-cancer patients.

Accordingly, when the expression levels of mRNA of two or more genes selected from the group consisting of VTN, NEDD4, CXCL5, LCN2, SYTL4, TNFR2, and SERPING1, or proteins encoded by two or more of the genes, are higher than those of a normal control group, it may be determined that radioresistance is present, and when the expression levels are lower, it may be determined that radioresistance is low or radiosensitivity is present. For example, when mRNA of two to seven genes selected from the group, and/or two to seven proteins encoded by the genes, are all highly expressed, it may be determined that radioresistance is high, and when the mRNA of two to seven genes and/or the two to seven proteins encoded by the genes are all lowly expressed, it may be determined that radioresistance is low.

Furthermore, when the expression levels of mRNA of two or more genes selected from the group consisting of VTN, NEDD4, CXCL5, LCN2, SYTL4, TNFR2, and SERPING1, or proteins encoded by two or more of the genes, are higher than those of a normal control group, it may be determined that the prognosis of radiotherapy for lung cancer is negative, and when the expression levels are lower, it may be determined that the prognosis of radiotherapy for lung cancer is positive. For example, when mRNA of two to seven genes selected from the group, and/or two to seven proteins encoded by the genes, are all highly expressed, it may be determined that a radiotherapy prognosis of lung cancer is negative, and when the mRNA of two to seven genes and/or the two to seven proteins encoded by the genes are all lowly expressed, it may be determined that a radiotherapy prognosis of lung cancer is positive.

Meanwhile, a composition for diagnosing radioresistance of lung cancer and a composition for predicting a radiotherapy prognosis of lung cancer according to the present disclosure may include an agent for measuring an expression level of mRNA of a VTN or NEDD4 gene or a protein encoded by the gene.

In the present disclosure, the term "radioresistance" refers to a state in which cells are not easily affected by radiation exposure and show little change in repair or proliferation when exposed to radiation. As a term opposite to the above radioresistance, "radiosensitivity" refers to a state in which cells are easily affected by radiation exposure and show changes in repair or proliferation when exposed to radiation.

In the present disclosure, the term "radiotherapy" refers to a cancer treatment that induces the death of cancer cells through a mechanism of inhibiting cell division by irradiating radiation to cause DNA damage in the cancer cells. The radiotherapy may be performed for curative, adjuvant, or palliative purposes in the treatment of solid tumors, and may be conducted alone, in combination with surgical treatment, or in combination therapy with chemotherapeutic agents or radiation sensitizers.

In the present disclosure, lung cancer may include small-cell lung cancer, non-small-cell lung cancer, lung adenocarcinoma, squamous cell carcinoma, and large-cell carcinoma.

In the present disclosure, the term "diagnosis" includes determining the susceptibility of a subject to a specific disease or disorder, determining whether a subject currently has a specific disease or disorder, determining the prognosis of a subject having a specific disease or disorder, or performing therametrics (for example, monitoring the condition of a subject to provide information on therapeutic efficacy).

In the present disclosure, the term "prognosis" refers to a prospect or preliminary assessment of the medical outcome of a disease and includes a positive prognosis and a negative prognosis. The positive prognosis includes improvement or stabilization of a disease, such as remission of the disease, tumor regression, long-term survival potential, or disease-free survival rate, and the negative prognosis includes progression or fatality of the disease, such as decreased survival rate, recurrence, tumor growth, metastasis, or drug resistance.

Furthermore, in the present disclosure, the term "prediction" refers to a medical inference, for example, anticipating in advance the course of a disease (such as disease progression, improvement, recurrence, tumor growth, drug resistance, possibility of death after treatment, or survival rate) or a responsiveness to a treatment method such as chemotherapy or radiotherapy.

Specifically, in the present disclosure, the diagnosis may be a diagnosis of radioresistance of lung cancer, and the prognosis prediction may be a prognosis prediction of radiotherapy of lung cancer.

In the present disclosure, the measurement of an mRNA expression level refers to a process of identifying the presence and degree of expression of the gene's mRNA in a biological sample for diagnosing radioresistance of lung cancer and/or predicting a prognosis of lung cancer radiotherapy, an agent for measuring the expression level of the mRNA may include a primer, a probe, or an anti-sense nucleotide that specifically binds to the gene or the mRNA, and the measurement of the mRNA expression level may be performed by one or more methods selected from the group consisting of RT-PCR, competitive RT-PCR, real-time RT-PCR, RNase protection assay (RPA), Northern blotting, and DNA chip analysis, but the present disclosure is not limited thereto.

In the present disclosure, the measurement of a protein expression level refers to a process of identifying the presence and degree of expression of the protein in a biological sample for diagnosing radioresistance of lung cancer and/or predicting a prognosis of lung cancer radiotherapy, an agent for measuring the expression level of the protein may include a polypeptide, a compound, an antibody, or an aptamer that may specifically bind to the protein, and the measurement of the protein expression level may be performed by one or more methods selected from the group consisting of Western blotting, enzyme linked immunosorbent assay (ELISA), radioimmunoassay (RIA), radioimmunodiffusion, Ouchterlony immunodiffusion, rocket immunoelectrophoresis, immunohistochemistry, dual luciferase reporter assay, immunoprecipitation assay, complement fixation assay, fluorescence activated cell sorter (FACS), and protein chip analysis, but the present disclosure is not limited thereto.

In the present disclosure, a "sample" may refer to a sample derived from a subject who has received radiotherapy or for whom a decision on whether to receive radiotherapy is required. For example, the term "sample" refers to a sample capable of specifying expression levels of mRNA of two or more genes selected from the group consisting of VTN, NEDD4, CXCL5, LCN2, SYTL4, TNFR2, and SERPING1, or proteins encoded by two or more of the genes, and may include cells, tissues, whole blood, serum, plasma, saliva, sputum, cerebrospinal fluid, or urine of a subject, but the present disclosure is not limited thereto.

In the present disclosure, a "subject" refers to an individual to be evaluated for radioresistance or the possibility of progression of lung cancer, or to be predicted for a prognosis of radiotherapy. The subject is not particularly limited as long as it is an animal in which lung cancer may develop, but may specifically be a mammal, for example, a human (Homo sapiens).

In the present disclosure, the term "expression level" may be used interchangeably with "expression signature" or "expression profile," and may include expression levels of mRNA of two or more genes selected from the group consisting of VTN, NEDD4, CXCL5, LCN2, SYTL4, TNFR2, and SERPING1, or proteins encoded by two or more of the genes. The expression level may include an increase or a decrease in expression, and the expression level may be measured from cancer cells isolated from a subject before and/or after radiotherapy. Meanwhile, the expression level may be identified by measuring an amount of mRNA of the gene and/or the protein in the sample.

In the present disclosure, the term "primer" refers to a short nucleic acid sequence having a free 3'-hydroxyl end, which can form base pairs with a complementary template and serves as a starting point for copying a template strand. The primer may initiate DNA synthesis in the presence of a reagent for a polymerization reaction (that is, DNA polymerase or reverse transcriptase) and four different nucleoside triphosphates, under appropriate buffer conditions and temperature. The primer of the present disclosure is a primer that may complementarily bind to the biomarker gene or mRNA of the present disclosure, and may be a sense or antisense nucleic acid having a nucleotide sequence of 7 to 50 bases, and may incorporate additional features that do not alter the essential property of the primer to serve as an initiation point for DNA synthesis. Meanwhile, the primer of the present disclosure may be chemically synthesized by using a phosphoramidite solid-support method or other well-known methods, and may be modified by using various means known in the art. Non-limiting examples of such modifications include methylation, capping, substitution with one or more analogs of natural nucleotides, and modifications between nucleotides, for example, modification to uncharged linkages (e.g., methyl phosphonate, phosphotriester, phosphoramidate, carbamate, etc.) or charged linkages (e.g., phosphorothioate, phosphorodithioate, etc.). The nucleic acid may contain one or more additional covalently bound residues, such as a protein (e.g., nuclease, toxin, antibody, signal peptide, or poly-L-lysine), an intercalator (e.g., acridine or psoralen), a chelating agent (e.g., metal, radioactive metal, iron, or oxidized metal), and an alkylating agent. In addition, the primer of the present disclosure may be modified by using a label capable of providing a detectable signal directly or indirectly, such as a radioactive isotope, a fluorescent molecule, or biotin.

In the present disclosure, the term "probe" refers to a nucleic acid that may complementarily bind to mRNA and is produced through enzymatic or chemical purification or synthesis processes, having a length of several to hundreds of bases. The probe of the present disclosure is a probe capable of complementarily binding to the biomarker gene or mRNA of the present disclosure, and may be labeled with a radioactive isotope or an enzyme to identify the presence or absence of mRNA, and may be designed and modified by known methods.

In the present disclosure, the term "antisense nucleotide" refers to DNA, RNA, or a derivative thereof containing a nucleic acid sequence complementary to the sequence of a specific mRNA, and binds to the complementary sequence within the mRNA and inhibits the translation of the mRNA into a protein. A sequence of the anti-sense nucleotide refers to a DNA or RNA sequence that is complementary to the mRNA of the biomarker gene of the present disclosure and that may bind to the mRNA. This may inhibit an essential activity of the mRNA in translation, translocation into the cytoplasm, maturation, or any other overall biological function. The antisense nucleotide may be synthesized in vitro by conventional methods and administered in vivo, or may be produced in vivo, and the antisense nucleotide usable in the present disclosure may be prepared according to methods known in the art, with reference to the nucleotide sequence and/or amino acid sequence of the biomarker gene and/or protein of the present disclosure.

In the present disclosure, the term "antibody" refers to a globulin-type protein that circulates in blood or lymph within the immune system of a living organism and reacts to an invading external substance, that is, an antigen, and means a protein that specifically binds to the antigen. For purposes of the present disclosure, the antibody refers to an antibody that specifically binds to the biomarker protein of the present disclosure, and may include polyclonal antibodies, monoclonal antibodies, and recombinant antibodies, and includes not only a complete form having two full-length light chains and two full-length heavy chains, but also functional fragments of an antibody molecule. The functional fragment of an antibody molecule refers to a fragment that retains at least an antigen-binding function, and examples thereof include Fab, F(ab'), F(ab')₂, and Fv. An antibody that specifically binds to the biomarker protein of the present disclosure may be produced by methods known to those skilled in the art, for example, by injecting the immunogenic biomarker protein into an external host. The external host may include mammals such as mice, rats, sheep, and rabbits, and the immunogen may be injected by intramuscular, intraperitoneal, or subcutaneous injection, and is generally administered together with an adjuvant to enhance antigenicity. Thereafter, blood may be periodically collected from the external host, and serum showing specificity to the antigen may be obtained, from which the antibody can be isolated.

In the present disclosure, the term "aptamer" is a single-stranded oligonucleotide, has a length of about 20 to 60 nucleotides, and refers to a nucleic acid molecule having a binding activity to a specific target molecule. The aptamer has various three-dimensional structures depending on its sequence, has high affinity for specific substances like an antigen-antibody reaction, and binds to a specific target molecule so that it may inhibit the activity of the specific target molecule. The aptamer may be RNA, DNA, a modified nucleic acid, or a mixture thereof, and may also be in a linear or circular form. In the present disclosure, the aptamer may specifically bind to the protein encoded by the biomarker gene of the present disclosure, and may be prepared by a person skilled in the art from a nucleotide sequence of the biomarker gene of the present disclosure by using known methods.

### 2. Kit for Diagnosing Radioresistance of Lung Cancer and Kit for Predicting a Prognosis of Lung Cancer Radiotherapy

Another aspect of the present disclosure provides a kit for diagnosing radioresistance of lung cancer.

Furthermore, another aspect of the present disclosure provides a kit for predicting a prognosis of radiotherapy for lung cancer.

The kit for diagnosing radioresistance of lung cancer according to the present disclosure includes, as an active ingredient, an agent for measuring expression levels of mRNA of two or more genes selected from the group consisting of VTN, CXCL5, NEDD4, LCN2, SYTL4, TNFR2, and SERPING1, or proteins encoded by two or more of the genes.

Furthermore, the kit for predicting a prognosis of radiotherapy for lung cancer according to the present disclosure includes, as an active ingredient, an agent for measuring expression levels of mRNA of two or more genes selected from the group consisting of VTN, CXCL5, NEDD4, LCN2, SYTL4, TNFR2, and SERPING1, or proteins encoded by two or more of the genes.

As described above in connection with the composition for diagnosing radioresistance of lung cancer or for predicting a prognosis of lung cancer radiotherapy, a description thereof will be omitted.

In the present disclosure, the term "kit" refers to a tool that may diagnose radioresistance of lung cancer or predict a prognosis of lung cancer radiotherapy by identifying expression levels of mRNA of two or more genes selected from the group consisting of VTN, CXCL5, NEDD4, LCN2, SYTL4, TNFR2, and SERPING1, or proteins encoded by two or more of the genes. The kit of the present disclosure may include an agent for measuring expression levels of mRNA of two or more genes selected from the group consisting of VTN, CXCL5, NEDD4, LCN2, SYTL4, TNFR2, and SERPING1, or proteins encoded by two or more of the genes, and specifically, primers, probes, and antisense nucleotides that specifically bind to the mRNA of the genes, or polypeptides, compounds, antibodies, and aptamers that specifically bind to the proteins, and may further include one or more other compositions, solutions, or devices suitable for the analytical method.

Furthermore, the kit may additionally include a user manual that describes optimal reaction conditions. The manual may include an instruction booklet in the form of a pamphlet or leaflet, a label attached to the kit, or a description on the surface of a package containing the kit, and may also include information disclosed or provided through an electronic medium such as the Internet.

In the present disclosure, the kit may be an RT-PCR kit, a microarray chip kit, a DNA kit, an ELISA kit, a protein chip kit, or a rapid kit.

### 3. Method for Providing Information for Diagnosing Radioresistance of Lung Cancer and Method for Providing Information for Predicting Prognosis of Lung Cancer Radiotherapy

Another aspect of the present disclosure provides a method for providing information for diagnosing radioresistance of lung cancer.

Furthermore, another aspect of the present disclosure provides a method for providing information for predicting a prognosis of lung cancer radiotherapy.

The method for providing information for diagnosing radioresistance of lung cancer according to the present disclosure includes (a) measuring, in a sample isolated from a subject, expression levels of mRNA of two or more genes selected from the group consisting of VTN, CXCL5, NEDD4, LCN2, SYTL4, TNFR2, and SERPING1, or proteins encoded by two or more of the genes, and (b) comparing the expression levels of the mRNA or proteins with expression levels of mRNA or proteins of a normal control group.

Furthermore, the method for providing information for predicting a prognosis of lung cancer radiotherapy according to the present disclosure includes (a) measuring, in a sample isolated from a subject, expression levels of mRNA of two or more genes selected from the group consisting of VTN, CXCL5, NEDD4, LCN2, SYTL4, TNFR2, and SERPING1, or proteins encoded by two or more of the genes, and (b) comparing the expression levels of the mRNA or proteins with expression levels of mRNA or proteins of a normal control group.

As described above in connection with the composition for diagnosing radioresistance of lung cancer or for predicting a prognosis of lung cancer radiotherapy, a description thereof will be omitted.

In the method for providing information for diagnosing radioresistance of lung cancer according to the present disclosure, (a) may include measuring an expression level of mRNA of the VTN or NEDD4 gene or a protein encoded by the gene.

Furthermore, in the method for providing information for predicting a prognosis of lung cancer radiotherapy according to the present disclosure, (a) may include measuring an expression level of mRNA of the VTN or NEDD4 gene or a protein encoded by the gene.

In the method for providing information for diagnosing radioresistance of lung cancer according to the present disclosure, when the expression levels of the mRNA or proteins are compared with expression levels of mRNA or proteins of a normal control group in (b), and the expression levels are higher than those of the normal control group, it may be determined that the lung cancer has radioresistance. The expression that the expression level is high may include that an expression level of a subject is at a similar level to, or increased by 1%, 2%, 3%, 4%, 5%, 10%, 20%, 30%, 40%, 50%, 60%, 70%, 80%, 90%, 100%, 200%, 300%, 400%, 500%, 600%, 700%, 800%, 900%, or 1000% or more compared with an expression level of a normal control group.

Furthermore, in the method for providing information for predicting a prognosis of radiotherapy of lung cancer according to the present disclosure, when the expression levels of the mRNA or proteins are compared with expression levels of mRNA or proteins of a normal control group in (b), and the expression levels are higher than those of the normal control group, it may be determined that a radiotherapy prognosis of the lung cancer is negative. The expression that the expression level is high may include that an expression level of a subject is at a similar level to, or increased by 1%, 2%, 3%, 4%, 5%, 10%, 20%, 30%, 40%, 50%, 60%, 70%, 80%, 90%, 100%, 200%, 300%, 400%, 500%, 600%, 700%, 800%, 900%, or 1000% or more compared with an expression level of a normal control group.

Meanwhile, the method for providing information for diagnosing radioresistance of lung cancer according to the present disclosure may further include (c) determining that the lung cancer has radioresistance when the measured expression levels of the mRNA or proteins are higher than those of the normal control group.

Furthermore, the method for providing information for predicting a prognosis of lung cancer radiotherapy according to the present disclosure may further include (c) determining that a radiotherapy prognosis of the lung cancer is negative when the measured expression levels of the mRNA or proteins are higher than those of the normal control group.

In a specific embodiment of the present disclosure, genes or combinations thereof having a P-value of 0.05 or less were identified by analyzing overall survival (OS) using TCGA LUAD (lung adenocarcinoma) and TCGA LUSC (lung squamous cell carcinoma) data, and hazard ratio (HR) values according to each gene or combination were identified. The HR indicates a risk, and a higher HR value in a high-expression group means that a survival rate of the subject decreases. When the HR is 1.2 or more, 1.3 or more, 1.4 or more, or 1.5 or more, it may be determined that a radiotherapy prognosis of the subject's lung cancer is negative.

Another aspect of the present disclosure provides a method for diagnosing radioresistance of lung cancer, including (a) measuring, in a sample isolated from a subject, expression levels of mRNA of two or more genes selected from the group consisting of VTN, CXCL5, NEDD4, LCN2, SYTL4, TNFR2, and SERPING1, or proteins encoded by two or more of the genes, and (b) comparing the expression levels of the mRNA or proteins with expression levels of mRNA or proteins of a normal control group.

Another aspect of the present disclosure provides a method for predicting a prognosis of radiotherapy of lung cancer, including (a) measuring, in a sample isolated from a subject, expression levels of mRNA of two or more genes selected from the group consisting of VTN, CXCL5, NEDD4, LCN2, SYTL4, TNFR2, and SERPING1, or proteins encoded by two or more of the genes, and (b) comparing the expression levels of the mRNA or proteins with expression levels of mRNA or proteins of a normal control group.

Another aspect of the present disclosure provides a use of an agent for measuring expression levels of mRNA of two or more genes selected from the group consisting of VTN, CXCL5, NEDD4, LCN2, SYTL4, TNFR2, and SERPING1, or proteins encoded by two or more of the genes, for diagnosing radioresistance of lung cancer.

Another aspect of the present disclosure provides a use of an agent for measuring expression levels of mRNA of two or more genes selected from the group consisting of VTN, CXCL5, NEDD4, LCN2, SYTL4, TNFR2, and SERPING1, or proteins encoded by two or more of the genes, for predicting a prognosis of lung cancer radiotherapy.

### [Advantageous Effects]

In the present disclosure, mRNA of the VTN, CXCL5, NEDD4, LCN2, SYTL4, TNFR2, and SERPING1 genes or proteins encoded by the genes have differential expression levels in radioresistant lung cancer cells compared with control lung cancer cells, and are closely correlated with reduced survival rates of patients with lung cancer. Accordingly, an agent for measuring an expression level of mRNA of a gene selected from the group consisting of VTN, CXCL5, NEDD4, LCN2, SYTL4, TNFR2, and SERPING1 of the present disclosure, or a protein encoded by the gene, may effectively diagnose radioresistance of lung cancer or predict a prognosis of lung cancer radiotherapy, and, through this, by determining whether to administer radiotherapy and an optimal radiotherapy dose, may improve an efficiency and therapeutic outcome of radiotherapy for lung cancer.

However, the effects of the present disclosure are not limited to those mentioned above, and other effects not mentioned will be clearly understood by those skilled in the art from the following description.

### [Description of Drawings]

FIG. 1a shows results of measuring radiation sensitivity for human lung cancer cell lines H1975, H2126, and H1650 (parental) and their respective radioresistance (RR) cell lines.
FIG. 1b shows results of measuring survival rates for human lung cancer cell lines H1975, H2126, and H1650 (parental) and their respective radioresistance (RR) cell lines.
FIG. 2 shows results of analyzing genes whose expression levels were increased or decreased after exposure to an 8-Gy dose of radiation in human lung cancer cell lines H1975, H2126, and H1650 (Par) and their respective radioresistance (RR) cell lines.
FIG. 3 shows results of analyzing changes in expression levels of proteins of radioresistance genes in human lung cancer cell lines H1975 and H2126 (Parent) and their respective radioresistance (RR) cell lines.
FIG. 4 shows results of analyzing the expression level of LCN2 using DESeq2 based on RNA-seq data (GSE81089) from 218 patients with non-small-cell lung cancer.
FIG. 5a shows Kaplan-Meier survival curves for survival rates of lung cancer patients according to expression levels of VTN, NEDD4, CXCL5, or LCN2.
FIG. 5b shows Kaplan-Meier survival curves for survival rates of lung cancer patients according to expression levels of VTN and NEDD4, or expression levels of CXCL5 and LCN2.
FIG. 5c shows Kaplan-Meier survival curves for survival rates of lung cancer patients according to expression levels of CXCL5, LCN2, and SERPING1, or expression levels of CXCL5, LCN2, VTN, and SYTL4.
FIG. 6 shows changes in cell survival rates measured after knocking down expression of radioresistance genes with siRNA and exposing human radioresistance lung cancer cell line H1975-RR to radiation.

### [Mode for Invention]

Hereinafter, the present disclosure will be described in detail with reference to examples.

However, the following examples are merely illustrative of the present disclosure, and the scope of the present disclosure is not limited to these examples.

### Example 1. Generation of Radioresistance Lung Cancer Cell Lines and Measurement of Radiation Sensitivity

Human lung cancer cell lines H1975, H2126, and H1650 were purchased from ATCC (American Type Culture Collection, USA) and maintained in RPMI-1640 medium supplemented with 10% fetal bovine serum (FBS) and 1% penicillin-streptomycin (10,000 U/ml) under conditions of 5% CO₂ at 37°C. Thereafter, radioresistance cell lines were established by exposing the cell lines to a total of 60 Gy of radiation, administered at 2 Gy per session, one to two times per week.

Cells that had completed radiation exposure were evaluated for radiation sensitivity by exposing them to radiation at respective doses and then culturing them for 10-14 days to assess colony-forming ability through a clonogenic assay. Specifically, each cell line was seeded at an appropriate cell number in a 60-mm dish, and after 24 hours had elapsed, the cells were exposed to radiation at respective doses and then cultured for 10 to 14 days under conditions of 5% CO₂ at 37°C. Thereafter, the formed cell colonies were washed with phosphate-buffered saline (PBS) and stained with a mixture of 100% methanol containing 1% methylene blue. The stained cell colonies (colonies containing 50 or more cells) were air-dried at room temperature and visually counted for each dish.

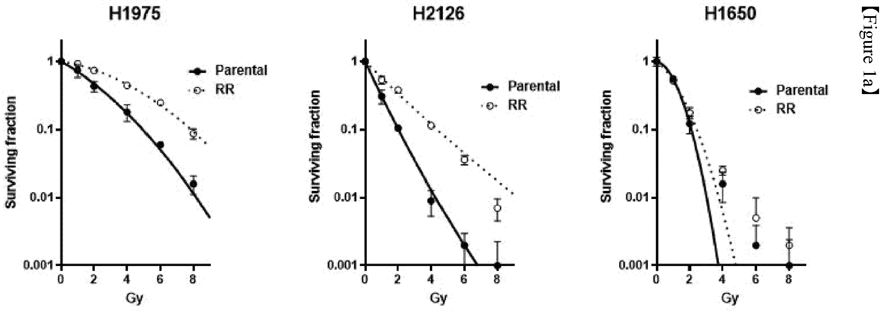

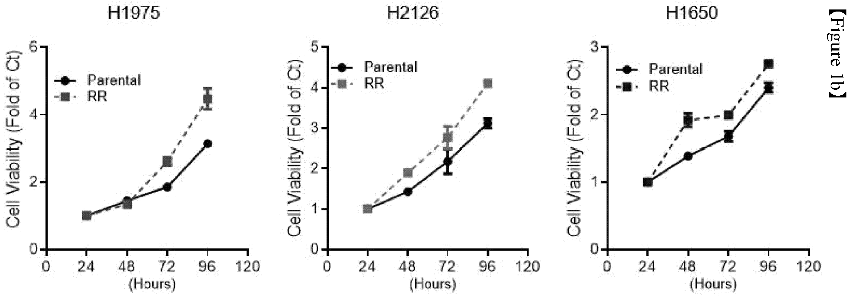

As a result, it was identified that the radioresistance (RR) cell lines exhibited higher colony-forming ability than the parental cell lines, indicating resistance to radiation-induced cell death (FIG. 1a). Furthermore, after culturing the cell lines for 24 to 96 hours and measuring cell survival rates by using an MTT assay, it was identified that cell growth rates were increased in the radioresistance cell lines compared to the parental cell lines (FIG. 1b).

### Example 2. Discovery of Radioresistance-Related Genes in Lung Cancer

To discover radioresistance-related genes in lung cancer, genes were selected based on transcriptome data analyzed from the radioresistance cell lines and their corresponding parental cell lines, and radiation responsiveness of each gene was measured by real-time reverse transcription polymerase chain reaction (real-time RT-PCR). The primer information used in the present example is as in Table 1 below.

**[Table 1]**

| **Target Sequence** | **Officia l Gene Name** | **Produc t length** | **Dir.** | **Sequence (5'->3')** |
|---|---|---|---|---|
| NM_000638.4 | VTN | 154 | Forward | TGG CTG TCC TTG TTC TCC AGT G (Sequence ID No. 8) |
| | | | Reverse | GTG TGC GAA GAT TGA CTC GGT AG (Sequence ID No. 9) |
| NM_002994.5 | CXCL 5 | 111 | Forward | CAG ACC ACG CAA GGA GTT CAT C (Sequence ID No. 10) |
| | | | Reverse | TTC CTT CCC GTT CTT CAG GGA G (Sequence ID No. 11) |
| NM_00128433 8.2 | NEDD 4 | 95 | Forward | CAG AAG AGG CAG CTT ACA AGC C (Sequence ID No. 12) |
| | | | Reverse | CTT CCC AAC CTG GTG GTA ATC C (Sequence ID No. 13) |
| NM_005564.5 | LCN2 | 112 | Forward | GTG AGC ACC AAC TAC AAC CAG C (Sequence ID No. 14) |
| | | | Reverse | GTT CCG AAG TCA GCT CCT TGG T (Sequence ID No. 15) |
| NM_00112989 6.3 | SYTL4 | 94 | Forward | GGA GAC AGA AGC AAA TCC GTC C (Sequence ID No. 16) |
| | | | Reverse | GGC GAT GTA ACT TCA CTA GGT GG (Sequence ID No. 17) |
| NM_001066.3 | TNFRS F1B | 102 | Forward | CGT TCT CCA ACA CGA CTT CAT CC (Sequence ID No. 18) |
| | | | Reverse | ACG TGC AGA CTG CAT CCA TGC T (Sequence ID No. 19) |
| NM_000062.3 | SERPI NG1 | 157 | Forward | GCA TCA AAG TGA CGA CCA GCC A (Sequence ID No. 20) |
| | | | Reverse | GTC TCT GTC AGT TCC AGC ACT G (Sequence ID No. 21) |
| NM_022551.3 | RPS18 | 107 | Forward | CAC GCC AGT ACA AGA TCC CA (Sequence ID No. 22) |

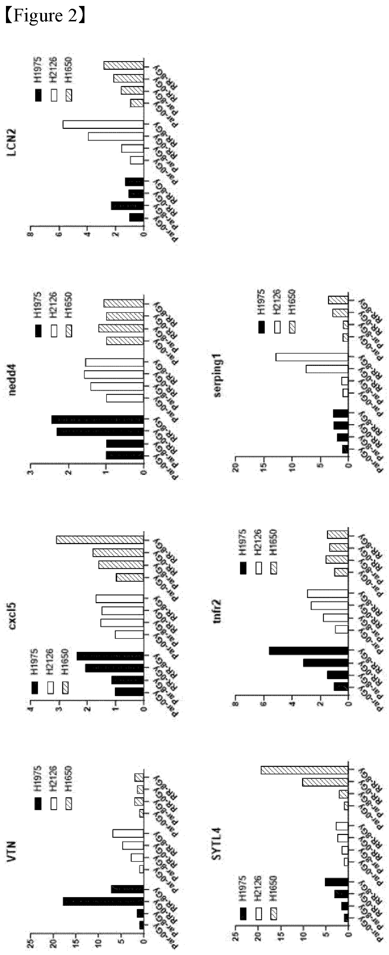

Thereafter, parental lung cancer cell lines (Par) of three types (H1975, H2126, and H1650) and their radioresistant lung cancer cell lines (RR) were irradiated with 0 or 8 Gy of radiation, and expression of each gene was measured 24 hours later. As a result, it was shown that VTN, CXCL5, NEDD4, LCN2, SYTL4, TNFR2, and SERPING1 were upregulated in the radioresistance cells compared to the parental cells, and that their expression levels were increased by radiation exposure not only in the parental cells but also in the radioresistance cells (FIG. 2).

### Example 3. Identification of Protein Expression Changes in Radiation-Responsive Factors

For the radioresistance genes identified in Example 2, to identify patterns of changes in protein expression levels in response to radiation exposure, Western blotting assays were performed by extracting proteins 24 hours after exposing the H1975 lung cancer cell line, the H2126 lung cancer cell line, and their respective radioresistance cell lines to an 8-Gy dose of radiation. The antibody information used in the present example is as in Table 2 below.

**[Table 2]**

| Primary Antibody | Manufacturer | Cat.No | Host | Size (kD) |
|---|---|---|---|---|
| VTN | Santa Cruz | sc-74484 | Mouse | 75 |
| CXCL5 | Invitrogen | 700656 | Rabbit | 8 |
| NEDD4 | Santa Cruz | sc-14428 | Goat | 120 |
| LCN2 | Invitrogen | PA5-79589 | Rabbit | 22 |
| SYTL4 | Invitrogen | PA5-35992 | Rabbit | 80-90 |
| TNFR2 | Invitrogen | MA5-32618 | Rabbit | 60 |
| SERPING1 | Invitrogen | PA5-31541 | Rabbit | 100 |
| ACTB | Sigma | A5316 | Mouse | 42 |

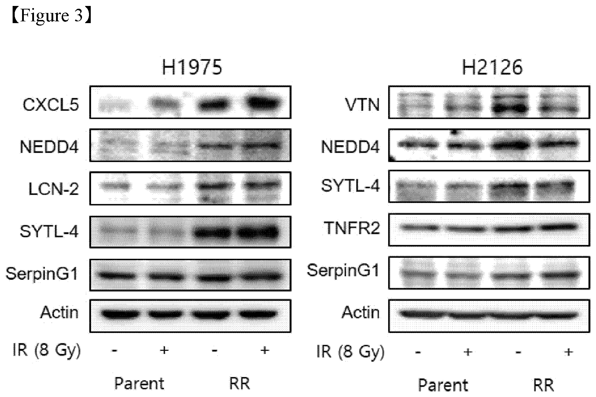

As a result of the analysis, it was shown that CXCL5, NEDD4, LCN2, SYTL4, and SERPING1 were significantly increased in the radioresistance cell line of the H1975 lung cancer cell line, and that VTN, NEDD4, LCN2, SYTL4, TNFR2, and SERPING1 were significantly increased in the radioresistance cell line of the H2126 lung cancer cell line, and it was shown that expression of CXCL5, LCN2, and TNFR2 was significantly increased by radiation exposure in both the parental and radioresistance cell lines (FIG. 3).

### Example 4. Analysis of Risk and Survival Curves According to Expression of Radioresistance Gene Panels in Lung Cancer Patients

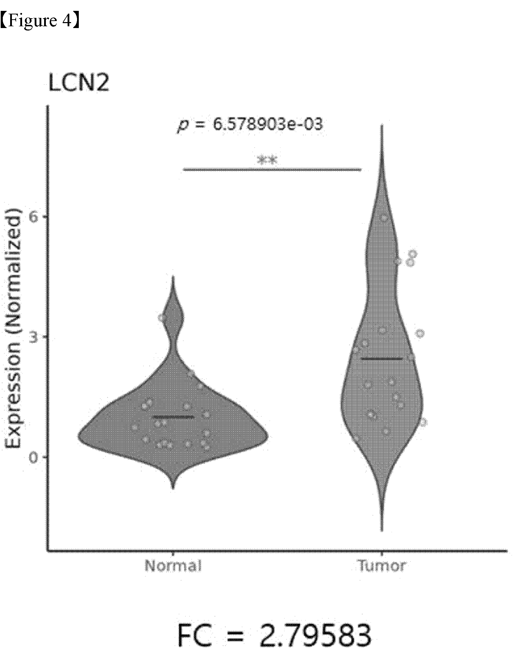

To identify expression patterns of the radioresistance genes derived in Example 2 in actual lung cancer patients, RNA-seq data (GSE81089) from 218 patients with non-small-cell lung cancer, which accounts for 80% of all lung cancer patients, were downloaded and analyzed using the DESeq2 program. As a result, it was shown that LCN2 was increased by 2.8-fold compared to the normal group (FIG. 4).

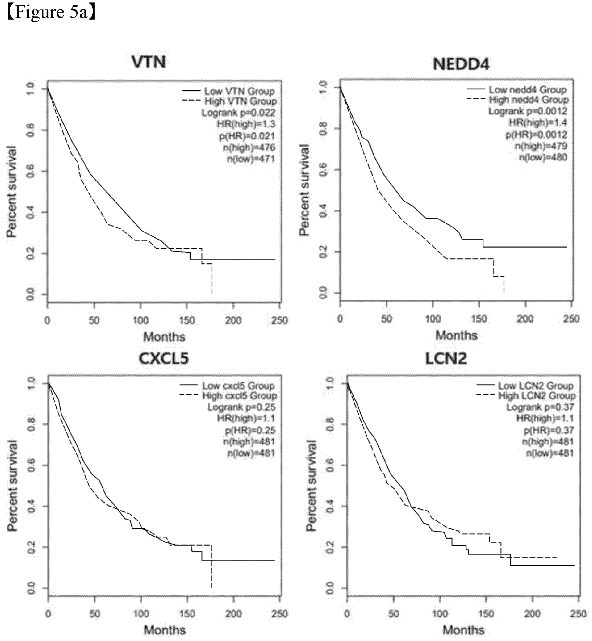

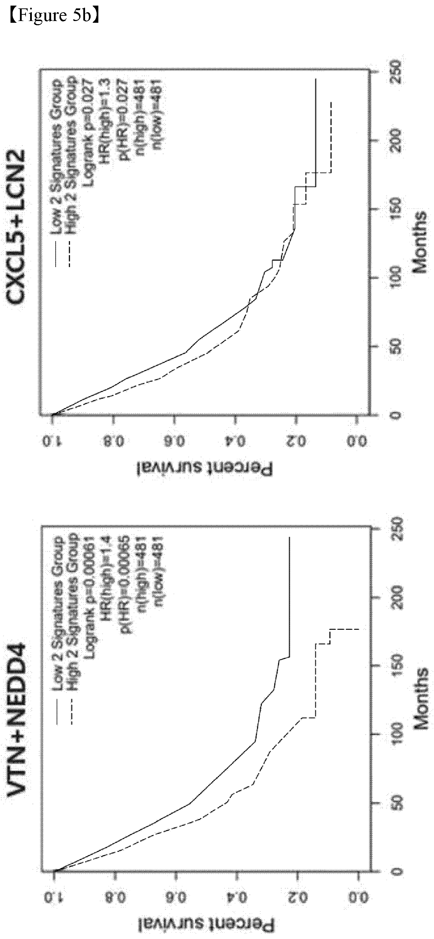

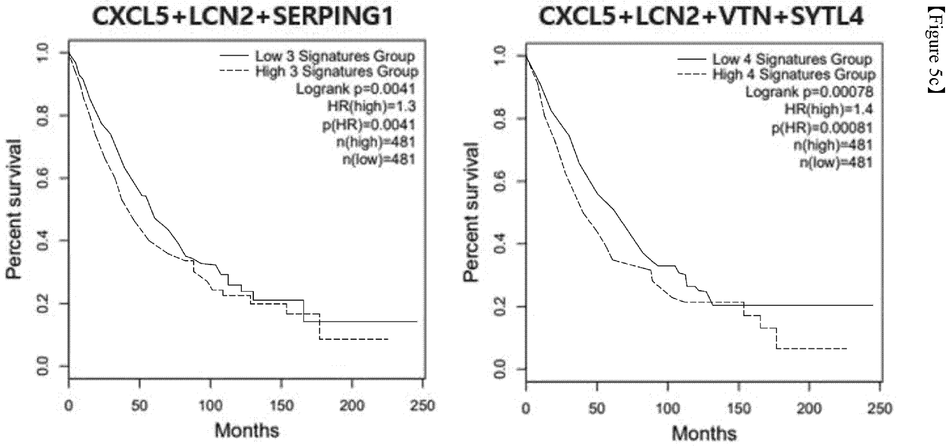

Thereafter, to analyze the effect of expression of the radioresistance genes derived in Example 2 on survival rates of lung cancer patients, TCGA (The Cancer Genome Atlas) data, which are publicly available genomic database resources, were analyzed using GEPIA2. In this case, to secure significant genes related to survival rates of lung cancer patients, overall survival (OS) was analyzed using TCGA LUAD (lung adenocarcinoma) and LUSC (lung squamous cell carcinoma) data, and genes or gene combinations with a P-value of 0.05 or less were identified, the results of which are presented in Table 3 below. In Table 3 below, the hazard ratio (HR) represents a risk level, and a higher HR value in the group with high gene expression indicates poorer patient survival. Furthermore, survival rates of lung cancer patients according to the expression of the above gene or gene combination were analyzed and visualized through Kaplan-Meier survival curves (FIGS. 5a to 5c).

**[Table 3]**

| **combination** | | | | | | **HR** | **P-value** |
|---|---|---|---|---|---|---|---|
| VTN | SerpinG1 | | | | | 1.2 | 0.033 |
| VTN | | | | | | 1.3 | 0.021 |
| VTN | NEDD4 | TNFR2 | | | | 1.3 | 0.0099 |
| VTN | NEDD4 | CXCL5 | | | | 1.3 | 0.0083 |
| VTN | NEDD4 | CXCL5 | LCN2 | | | 1.4 | 0.0031 |
| VTN | NEDD4 | SerpinG1 | | | | 1.4 | 0.0027 |
| VTN | NEDD4 | SerpinG1 | SYTL4 | | | 1.4 | 0.00098 |
| NEDD4 | CXCL5 | | | | | 1.3 | 0.016 |
| NEDD4 | SerpinG1 | | | | | 1.3 | 0.015 |
| NEDD4 | | | | | | 1.4 | 0.0012 |
| NEDD4 | VTN | | | | | 1.4 | 0.00065 |
| NEDD4 | SYTL4 | | | | | 1.4 | 0.00018 |
| CXCL5 | LCN2 | | | | | 1.3 | 0.027 |
| CXCL5 | LCN2 | SYTL4 | | | | 1.3 | 0.0099 |
| CXCL5 | LCN2 | NEDD4 | | | | 1.3 | 0.0053 |
| CXCL5 | LCN2 | VTN | | | | 1.3 | 0.0041 |
| CXCL5 | LCN2 | SerpinG1 | | | | 1.3 | 0.0041 |
| CXCL5 | LCN2 | SerpinG1 | TNFR2 | | | 1.3 | 0.0034 |
| CXCL5 | LCN2 | VTN | NEDD4 | | | 1.4 | 0.0031 |
| CXCL5 | LCN2 | VTN | TNFR2 | | | 1.4 | 0.0029 |
| CXCL5 | LCN2 | VTN | SerpinG1 | | | 1.4 | 0.0017 |
| CXCL5 | LCN2 | VTN | SYTL4 | | | 1.4 | 0.00081 |
| CXCL5 | LCN2 | SerpinG1 | NEDD4 | | | 1.4 | 0.00044 |

As a result of the analysis, the risk for lung cancer patient survival associated with increased VTN expression was 1.3, the risk associated with increased NEDD4 expression was 1.4, and the risk associated with the simultaneous increase in expression of the VTN and NEDD4 genes was 1.4. Meanwhile, the risk associated with increased expression of CXCL5 or LCN2 was 1.1, but it was not statistically significant, whereas the simultaneous increase in expression of the CXCL5 and LCN2 genes showed a risk of 1.3, indicating a significant decrease in overall survival. It was shown that when the three-gene combination of CXCL5/LCN2/SERPING1 or the four-gene combinations of CXCL5/LCN2/VTN/SYTL4 or CXCL5/LCN2/SERPING1/NEDD4 were considered, the risk increased to 1.3, 1.4, and 1.4, respectively.

### Example 5. Analysis of Cell Viability Following Knockdown of Radiation-Responsive Genes in Human Lung Cancer Radioresistance Cells

To identify changes in a survival rate of radioresistance lung cancer cells when expression of the radioresistance genes derived from Example 2 was knocked down, expression of the radioresistance genes was knocked down by using siRNA, and the radioresistance H1975 lung cancer cell line (H1975-RR) was exposed to radiation, after which a change in the survival rate was measured.

Specifically, each siRNA was transfected into the H1975-RR cells using Lipofectamine^{™} RNAiMAX transfection reagent (Thermo Fisher Scientific, USA) in a 96-well plate, followed by radiation exposure at a dose of 8 Gy. Thereafter, after 48 hours had elapsed, cell survival rate was measured by treating the cells with CCK-8 (DOJINDO, Japan) reagent. The siRNA information used in the present example is as in Table 4 below.

**[Table 4]**

| **Official Gene Name** | **NCBI Gene ID** | **Direction** | **Sequence (5'->3')** |
|---|---|---|---|
| Scramble RNA | | Sense | CCUCGUGCCGUUCCAUCAGGUAG (SEQ ID No: 23) |
| | | Anti-sense | CUACCUGAUGGAACGGCACGAGG (SEQ ID No: 24) |
| VTN | 7448 | Sense | GUAGUCAGUACUGGCGCUUUG (SEQ ID No: 25) |
| | | Anti-sense | CAAAGCGCCAGUACUGACUAC (SEQ ID No: 26) |
| NEDD4 | 4734 | Sense | AGUGCUACUCGCAGCUAUUUA (SEQ ID No: 27) |
| | | Anti-sense | UAAAUAGCUGCGAGUAGCACU (SEQ ID No: 28) |
| CXCL5 | 6374 | Sense | UGAAUUGUAGGUGACUAUUAU (SEQ ID No: 29) |
| | | Anti-sense | AUAAUAGUCACCUACAAUUCA (SEQ ID No: 30) |
| LCN2 | 3934 | Sense | UGUCCCAAUCGACCAGUGUAU (SEQ ID No: 31) |
| | | Anti-sense | AUACACUGGUCGAUUGGGACA (SEQ ID No: 32) |
| SERPING1 | 710 | Sense | GCCAAGUGGAAGACAACAUUU (SEQ ID No: 33) |
| | | Anti-sense | AAAUGUUGUCUUCCACUUGGC (SEQ ID No: 34) |

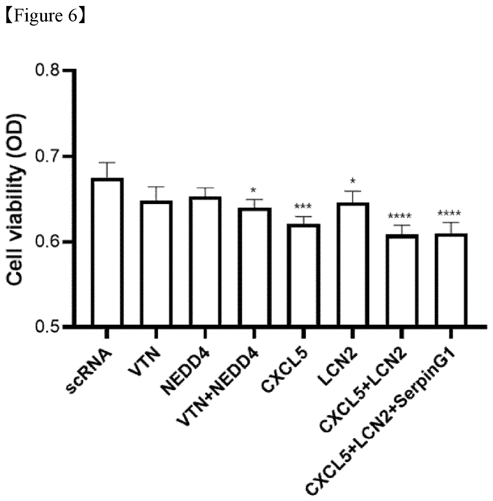

As a result, it was shown that, in the radioresistance lung cancer cell line, cell death induced by radiation exposure was significantly increased by knock-down of LCN2 or CXCL5 alone, compared to the scramble RNA (scRNA) group treated with a non-specific sequence as a control. Furthermore, although cell death induced by knock-down of VTN or NEDD4 alone was not significant compared to the control group, simultaneous knock-down of VTN and NEDD4 caused a significant decrease in lung cancer cell survival rate upon radiation exposure. Moreover, compared to knock-down of LCN2 alone, simultaneous knock-down of LCN2/CXCL5 or LCN2/CXCL5/SERPING1 showed more significant radiation-induced cell death (FIG. 6). Through this, it was identified that radioresistance of lung cancer may be overcome by knock-down of various gene combinations, and that cell death may be increased.

Through these experiments, it was identified that resistance to radiotherapy and prognosis of radiotherapy may be effectively predicted by measuring expression levels of VTN, CXCL5, NEDD4, LCN2, SYTL4, TNFR2, and SERPING1. Accordingly, it is expected that cancer may be treated more effectively when radiotherapy is combined while identifying the activity of the above radioresistance gene panel.

The representative examples of the present disclosure have been described above by way of example, but the scope of the present disclosure is not limited to the specific examples described above, and those skilled in the art will be able to make appropriate modifications within the scope set forth in the claims of the present application.

## Claims

1. A composition for diagnosing radioresistance of lung cancer, the composition comprising: an agent for measuring expression levels of mRNA of two or more genes selected from the group consisting of VTN, CXCL5, NEDD4, LCN2, SYTL4, TNFR2, and SERPING1 genes, or expression levels of proteins encoded by two or more of the genes.

2. The composition of claim 1, wherein the composition comprises an agent for measuring an expression level of mRNA of a CXCL5, LCN2, VTN, or NEDD4 gene, or an expression level of a protein encoded by the gene.

3. The composition of claim 1, wherein the agent for measuring the expression level of the mRNA of the gene is one or more selected from the group consisting of a primer, a probe, and an antisense nucleotide that specifically binds to the mRNA of the gene.

4. The composition of claim 1, wherein the agent for measuring the expression level of the protein is one or more selected from the group consisting of polypeptides, compounds, antibodies, and aptamers that specifically bind to the protein.

5. A kit for diagnosing radioresistance of lung cancer, the kit comprising: the composition of any one of claims 1 to 4.

6. The kit of claim 5, wherein the kit is an RT-PCR kit, a microarray chip kit, a DNA kit, an ELISA kit, a protein chip kit, or a rapid kit.

7. A method for providing information for diagnosing radioresistance of lung cancer, the method comprising:
(a) measuring, in a sample isolated from a subject, an expression level of mRNA of two or more genes selected from the group consisting of VTN, CXCL5, NEDD4, LCN2, SYTL4, TNFR2, and SERPING1 genes, or an expression level of proteins encoded by two or more of the genes; and
(b) comparing the expression levels of the mRNA or the proteins with expression levels of mRNA or proteins of a normal control group.

8. The method of claim 7, wherein (a) comprises measuring an expression level of mRNA of a CXCL5, LCN2, VTN, or NEDD4 gene, or an expression level of a protein encoded by the gene.

9. A composition for predicting a prognosis of radiotherapy of lung cancer, the composition comprising: an agent for measuring expression levels of mRNA of two or more genes selected from the group consisting of VTN, CXCL5, NEDD4, LCN2, SYTL4, TNFR2, and SERPING1 genes, or expression levels of proteins encoded by two or more of the genes.

10. The composition of claim 9, wherein the composition comprises an agent for measuring an expression level of mRNA of a CXCL5, LCN2, VTN, or NEDD4 gene, or an expression level of a protein encoded by the gene.

11. The composition of claim 9, wherein the agent for measuring the expression level of the mRNA of the gene is one or more selected from the group consisting of a primer, a probe, and an antisense nucleotide that specifically binds to the mRNA of the gene.

12. The composition of claim 9, wherein the agent for measuring the expression level of the protein is one or more selected from the group consisting of antibodies and aptamers that specifically bind to the protein.

13. A kit for predicting a prognosis of radiotherapy of lung cancer, the kit comprising: the composition of any one of claims 9 to 12.

14. The kit of claim 13, wherein the kit is an RT-PCR kit, a microarray chip kit, a DNA kit, an ELISA kit, a protein chip kit, or a rapid kit.

15. A method for providing information for predicting a prognosis of radiotherapy of lung cancer, the method comprising:
(a) measuring, in a sample isolated from a subject, expression levels of mRNA of two or more genes selected from the group consisting of VTN, CXCL5, NEDD4, LCN2, SYTL4, TNFR2, and SERPING1 genes, or expression levels of proteins encoded by two or more of the genes; and
(b) comparing the expression levels of the mRNA or the proteins with expression levels of mRNA or proteins of a normal control group.

16. The method of claim 15, wherein (a) comprises measuring an expression level of mRNA of a CXCL5, LCN2, VTN, or NEDD4 gene, or an expression level of a protein encoded by the gene.
